Europäisches Patentamt

European Patent Office

Office européen des brevets

(11) Publication number: **0 408 462 B1**

# EUROPEAN PATENT SPECIFICATION

(45) Date of publication of patent specification: **21.06.95** (51) Int. Cl.⁶: **B01D 39/16**

(21) Application number: **90402035.1**

(22) Date of filing: **13.07.90**

(54) **Filter material for seizure of leukocytes and method for production thereof.**

(30) Priority: **14.07.89 JP 180277/89**
    **25.06.90 JP 166350/90**

(43) Date of publication of application:
    **16.01.91 Bulletin 91/03**

(45) Publication of the grant of the patent:
    **21.06.95 Bulletin 95/25**

(84) Designated Contracting States:
    **BE DE FR GB IT NL SE**

(56) References cited:
    **EP-A- 0 217 698**
    **EP-A- 0 331 174**
    **EP-A- 0 378 684**
    **DE-A- 3 342 823**
    **US-A- 4 100 238**

    **PATENT ABSTRACTS OF JAPAN vol. 12, no. 338 (C-527)(3185) 12 September 1988PATENT ABSTRACTS OF JAPAN vol. 13, no. 278 (C-611)(3626) 26 June 1989**

    **PATENT ABSTRACTS OF JAPAN vol. 12, no. 198 (C-502)(3045) 8 June 1988**

(73) Proprietor: **TERUMO KABUSHIKI KAISHA**
    **44-1 Hatagaya 2-chome**
    Shibuya-ku
    Tokyo (JP)

(72) Inventor: **Tatebe, Ken, c/o Terumo Kabushiki Kaisha**
    **1500, Inokuchi,**
    **Nakai-cho**
    **Ashigarakami-gun,**
    **Kanagawa-ken (JP)**
    Inventor: **Seita, Yukio, c/o Terumo Kabushiki Kaisha**
    **1500, Inokuchi,**
    **Nakai-cho**
    **Ashigarakami-gun,**
    **Kanagawa-ken (JP)**
    Inventor: **Yamashita, Shuzo, c/o Terumo Kabushiki Kaisha**
    **1500, Inokuchi,**
    **Nakai-cho**
    **Ashigarakami-gun,**
    **Kanagawa-ken (JP)**
    Inventor: **Nemoto, Yasushi, c/o Terumo Kabushiki Kaisha**
    **1500, Inokuchi,**
    **Nakai-cho**
    **Ashigarakami-gun,**
    **Kanagawa-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Higuchi, Yasuyuki, c/o Terumo Kabushiki Kaisha**
**1500, Inokuchi,**
**Nakai-cho**
**Ashigarakami-gun,**
**Kanagawa-ken (JP)**


(74) Representative: **Gillard, Marie-Louise et al**
**Cabinet Beau de Loménie**
**158, rue de l'Université**
**F-75340 Paris Cédex 07 (FR)**

**Description**

BACKGROUND OF THE INVENTION

Field of the Invention:

This invention relates to a filter material for the seizure of leukocytes and a method for the production thereof. More particularly, it relates to a leukocyte-seizing filter material exhibiting a stable ability to seize leukocytes and having no possibility of admitting foreign matter and a method for the production thereof. It relates further to a leukocyte-seizing filter material sterilizable with high-pressure steam and a method for the production thereof.

Description of the Prior Art:

A long time has passed since the form of blood transfusion changed from the conventional whole blood transfusion to the component transfusion using only the component blood required by a patient. The degree of purity which is possessed by the fractionated blood component forms an important question for the component blood transfusion. Heretofore, the blood secured by donation has been centrifugally separated into concentrated red blood corpuscles (CRC), platelet concentrated plasma (PC), and platelet deficient blood plasma (PPP). The concentrated red blood corpuscles, as a componential preparation of red blood corpuscles, is widely used for componential blood transfusion to patients in need of red blood corpuscles. Since the concentrated red blood corpuscles copiously contain leukocytes, the idea of regarding them as the so-called whole blood has been finding widespread recognition. The fact that because of the transfusion of concentrated red blood corpuscles, a patient in need of only red blood corpuscles is compelled to take in a large amount of leukocytes as accompanied by red blood corpuscles has been arousing concern. The leukocytes which are contained in a red blood fraction as in the concentrated red blood corpuscles must be removed from the red blood fraction to the fullest possible extent from the standpoint of preventing the transfusion from manifesting adverse side effects due to the leakage of leukocytes. Numerous devices have been developed to date for the purpose of this prevention. As ways of heightening the purity of the preparation of red blood corpuscles, the method of gravitational centrifugal separation which makes effective use of the difference in specific gravity among different species of blood corpuscles, the method of seizure resorting to the action of adhesion arising from the viscosity of blood corpuscles, and the method of separation of leukocytes by the use of a red blood corpuscle coagulant have been put to use.

Among other methods mentioned above, the method using a material capable of seizing blood corpuscles has been finding popular acceptance because of high efficiency manifested in the removal of leukocytes and great simplicity of operation. In the method using a material for seizure, the operation of washing and recovering as with physiological saline solution for the purpose of ensuring efficient seizure of leukocytes and, at the same time, improving the yield of red blood corpuscles is required to avoid inducing separation of seized leukocytes. As materials for the seizure, fibers of natural cellulose, polyesters, polyamides, polyacrylonitrile, and glass, for example which are produced in an extremely small diameter and are packed in their unmodified form in a column or further fabricated as into non-woven fabrics are used in most cases.

In the method described above, when the fibers are packed by themselves in the column, uniform packing of the fibers is difficult to attain and consumes time and labor. The completed column, depending on the manner in which the fibers are packed therein, has a fair possibility of giving rise to the phenomenon of channeling while in use. When the packing density of fibers in the column is increased so as to ensure thoroughness of the seizure of leukocytes, the filtration time is unduly extended. The column has a further possibility that the fibers will leak from the column because they are not thoroughly intertwined. Where the fibers are further fabricated into a non-woven fabric, this material for seizure does not readily entail the problems mentioned above but nevertheless suffers from the disadvantage that the texture of the fabric is apt to be clogged with seized blood corpuscles.

Various ideas of using porous substances in materials for seizure in the place of such fibers as mentioned above have been proposed.

JP-B-61-39,060(1986), for example, proposes the use of a porous material containing continuous pores having an average diameter in the range of 25 to 60 $\mu$m. The technical idea of this invention resides in effecting seizure of such cells as monocytes and granulocytes by taking advantage of the high viscosity these cells possess as compared with other blood cells. Thus, it does not deserve to be called a fully satisfactory mesure for fulfilling the desire to attain thorough seizure for removal of all of the leukocytes inclusive of lymphocytes.

JP-B-63-26,089(1988) discloses the use of a porous material containing continuous pores having an average diameter in the range of 5 to 20 $\mu$m. The technical idea of this invention is claimed to accomplish seizure of leukocytes owing to the viscosity of leukocytic cells coupled with the filtration through the pores. This technical idea consists solely in defining the size of the pores in the porous material. The present inventors have prepared various porous materials possessing pores of the same diameter as mentioned above and different magnitudes of porosity and bubble point from those of the invention under discussion and tested them for separation of leukocytes, to find that effective separation of leukocytes fails in many cases and the porous materials show virtually no satisfactory ability to seize leukocytes unless they have a fairly large thickness, specifically, a thickness of at least 10 mm. When the material for seizure made of a porous substance has a large wall thickness of not less than 10 mm, it entails the disadvantage that it is apt to be clogged with seized blood cells and handicapped by increase of the priming volume and elongation of the filtration time.

As a filter material for seizure of leukocytes, the use of a porous polyvinyl formal of a three-dimensional reticularly continuous texture having continuous open pores of an average diameter in the range of 5 to 60 $\mu$m has been proposed [JP-A-1-75,014(1989)]. Unlike the porous material having only the pore diameter controlled as described above, this porous material made of polyvinyl formal can be expected to attain relatively efficient removal of leukocytes cells even with a small wall thickness. A desire has been expressed for the leukocyte-seizing filters of this class to attain growth in the future in the in-line type having a filter incorporated in a blood bag system. Generally, the blood bag system is formed by having a medical preparation contained in a blood bag made of a flexible resin such as, for example, vinyl chloride resin. For the leukocyte-seizing filter to be used in the in-line type, therefore, it is required to be capable of withstanding the high-pressure steam to be used for sterilization. The filter material formed of the aforementioned porous polyvinyl formal substance is incapable of withstanding the sterilization with the high-pressure steam and, therefore, is not applicable to the use in the in-line type.

A seizing material exhibiting a stable quality enough for the leukocyte-seizing filter remains yet to be developed.

An object of this invention, therefore, is to provide a novel leukocyte-seizing filter and a method for the production thereof.

Another object of this invention is to provide a leukocyte-seizing filter possessing a prominent and stable ability to seize leukocytes and effect efficient separation of leukocytes from blood and a method for the production thereof.

Yet another object of this invention is to provide a leukocyte-seizing filter capable of safely removing leukocytes without entailing the possibility of leaking any foreign matter and a method for the production thereof.

A further object of this invention is to provide a leukocyte-seizing filter material sterilizable with high-pressure steam and a method for the production thereof.

SUMMARY OF THE INVENTION

The objects described above are accomplished by a leukocyte-seizing filter material formed of a three-dimensional reticularly continuous porous material comprising at least one polymeric substance and possessing an average pore diameter in the range of 1 to 60 $\mu$m, a specific surface area in the range of 0.5 to 10 m$^2$/g, a porosity in the range of 30 to 95%, a bubble point in the range of 0.08 to 0.40 kg/cm$^2$, and a wall thickness in the range of 0.3 to 9.0 mm and allowed to be sterilized with high-pressure steam.

This invention further discloses a leukocyte-seizing filter material, wherein the average pore diameter is in the range of 2 to 50 $\mu$m, preferably 5 to 20 $\mu$m, the specific surface area in the range of 1 to 5 m$^2$/g, preferably 1.5 to 3.0 m$^2$/g, the porosity in the range of 50 to 95%, preferably 60 to 95%, the bubble point in the range of 0.13 to 0.27 kg/cm$^2$, and the wall thickness in the range of 0.5 to 5.0 mm, preferably 0.5 to 3.0 mm. This invention also discloses a leukocyte-seizing filter material, wherein the polymeric substance is one member selected from the group consisting of polyurethane, polyvinylidene fluoride, polysulfones, polyether sulfones, polyesters, poly(meth)acrylates, butadiene-acrylonitrile copolymers, polyamides, polyether polyamide block copolymers, and ethylene-vinyl alcohol copolymers. This invention also discloses a leukocyte-seizing filter material, wherein the polymeric substance is polyurethane.

The objects described above are further accomplished by a method for the production of a leukocyte-seizing filter material comprising preparing a raw material composition comprising at least one polymeric substance, a good solvent for the polymeric substance, and at least one pore-forming agent soluble or swellable in a poor solvent compatible with the good solvent, extrusion molding the raw material composition in a prescribed shape with an extruding device, and then leading the extrusion molded composition into and immersing in a bath formed mainly of a poor solvent relative to the polymeric substance thereby gelling the extrusion molded composition and, at the same time, allowing the pore-forming agent to be dissolved into the poor solvent and consequently effecting removal thereof from the shaped composition.

They are further accomplished by a method for the production of a leukocyte-seizing filter material comprising preparing a raw material composition comprising at least one polymeric substance, a good solvent for the polymeric substance, and at least one pore-forming agent soluble or swellable in a poor solvent compatible with the good solvent, applying a coating of the composition on the surface of a substrate, and leading the coated substrate into and immersing in a bath formed mainly of a poor solvent relative to the polymeric substance thereby gelling the applied coating and, at the same time, allowing the pore-forming agent to be dissolved into the poor solvent and consequently effecting removal thereof from the coated substrate.

This invention further discloses a method for the production of a leukocyte-seizing filter material, wherein the polymeric substance is one member selected from the group consisting of polyurethane, polyvinylidene fluoride, polysulfones, polyether sulfones, polyesters, poly(meth)acrylates, butadiene-acrylonitrile copolymers, polyamides, polyether polyamide block copolymers, and ethylene-vinyl alcohol copolymers. This invention further discloses a method for the production of a leukocyte-seizing filter material, wherein the polymeric substance is polyurethane. A method for the production of a leukocyte-seizing filter material, wherein the pore-forming agent is a water-soluble compound. A method for the production of a leukocyte-seizing filter meterial, wherein the pore-forming agent is a water-soluble polymeric compound. A method for the production of a leukocyte-seizing filter material, wherein the pore-forming agent is one member selected from the group consisting of polyvinyl alcohol, polyvinyl pyrrolidone, methyl cellulose, polyethers, polysaccharides, polyacrylic acid and salts thereof, and polyacrylamides.

The objects described above are also accomplished by a leukocyte-seizing filter material having formed on the surface of a porous substrate a layer of a three-dimensional reticularly continuous porous material comprising at least one polymeric substance and possessing an average pore diameter in the range of 1 to 60 $\mu$m, a specific surface area in the range of 0.5 to 10 $m^2/g$, a porosity in the range of 30 to 95%, a bubble point in the range of 0.08 to 0.30 $kg/cm^2$, a wall thickness in the range of 0.1 to 9.0 mm, and a surface pore ratio in the range of 6 to 90 %.

This invention further discloses a leukocyte-seizing filter material, wherein the average pore diameter is in the range of 2 to 50 $\mu$m, preferably 5 to 20 $\mu$m, the specific surface area in the range of 1 to 5$m^2/g$, preferably 1.5 to 3.0 $m^2/g$, the porosity in the range of 50 to 95%, preferably 60 to 95%, the bubble point in the range of 0.13 to 0.27 $kg/cm^2$, the wall thickness in the range of 0.3 5.0 mm, preferably 0.5 to 3.0 mm, and the surface pore ratio in the range of 10 to 85 %, preferably 13 to 80 %. This invention also discloses a leukocyte-seizing filter material, wherein the polymeric substance is one member selected from the group consisting of polyurethane, polyvinylidene fluoride, polysulfones, polyether sulfones, polyesters, poly(meth)acrylates, butadiene-acrylonitrile copolymers, polyamides, polyether polyamide block copolymers, and ethylene-vinyl alcohol copolymers. This invention also discloses a leukocyte-seizing filter material, wherein the polymeric substance is polyurethane.

The objects described above are further accomplished by a method for the production of a leukocyte-seizing filter material characterized by preparing a raw material composition comprising at least one polymeric substance, a good solvent for the polymeric substance, and at least one pore-forming agent soluble or swellable in a poor solvent compatible with the good solvent, applying a coating of the composition on the surface of a porous substrate, leading the coated substrate into and immersing in, a bath containing a poor solvent and a good solvent relative to the polymeric substance thereby effecting solidification of the coating, and thereafter allowing the pore-forming agent to be dissolved into the poor solvent and consequently effecting removal thereof from the coated substrate.

This invention also discloses a method for the production of a leukocyte-seizing filter material, wherein the polymeric substance is one member selected from the group consisting of polyurethane, polyvinylidene fluoride, polysulfones, polyether sulfones, polyesters, poly(meth)acrylates, butadiene-acrylonitrile copolymers, polyamides, polyether polyamide block copolymers, and ethylene-vinyl alcohol copolymers. This invention also discloses a method for the production of a leukocyte-seizing filter material, wherein the pore-forming agent is a water-soluble compound. This invention also discloses a method for the production of a leukocyte-seizing filter material, wherein the pore-forming agent is a water-soluble polymeric compound. This invention also discloses a method for the production of a leukocyte-seizing filter material, wherein the pore-forming agent is one member selected from the group consisting of polyvinyl alcohol, polyvinyl pyrrolidone, methyl cellulose, polyethers, polysaccharides, polyacrylic acid and salts thereof, and polyacrylamides.

The filter material of this invention is constructed as described above. It, therefore, possesses a prominent and stable ability to seize leukocytes, permits efficient removal of leukocytic component by a simple procedure from a leukocyte-suspending liquid such as blood and concentrated red blood corpuscles, attains the removal of leukocytes safely without entailing the possibility of the filter material developing such adverse phenomena as clogging and channeling while in service and shedding fragments and consequently defiling the removed leukocytes, and permits supply of a red blood fraction with high purity safely as for component transfusion, for example. Further, since the filter material is sterilizable with high-pressure steam, it can be advantageously utilized in the in-line type apparatus. Moreover, since the filter material of this invention has an extremely small wall thickness, the priming volume is small, the clogging otherwise possibly entailed when the leukocyte suspension is caused to flow by virtue of the difference in level is avoided, and the filtering time is short.

Further, in the present invention, when the polymeric substance is one member selected from the group mentioned above, preferably polyurethane, the filter material exhibits stabler characteristic properties after it has been sterilized with high-pressure steam and, consequently, finds utility in a still wider range of applications.

This invention is directed to a method as described above and, therefore, permits easy production of a leukocyte-seizing filter material possessing such outstanding properties as described above.

In the method of this invention for the production of a leukocyte-seizing filter material, when the polymeric substance is one member selected from the group mentioned above, particularly polyurethane, and the pore-forming agent is a water-soluble compound, preferably one member selected from the group consisting of polyvinyl alcohol, polyvinyl pyrrolidone, methyl cellulose, polyethers, polysaccharides, polyacrylic acid and salts thereof, and polyacrylamides, the produced leukocyte-seizing filter material acquires still better characteristic properties.

This invention is also directed to a leukocyte-seizing filter material which has formed on the surface of a porous substrate a layer of a three-dimensional reticularly continuous porous material comprising at least one polymeric substance and possessing an average pore diameter in the range of 1 to 60 $\mu$m, a specific surface area in the range of 0.5 to 10 m$^2$/g, a porosity in the range of 30 to 95%, a bubble point in the range of 0.08 to 0.30 kg/cm$^2$, a wall thickness in the range of 0.1 to 9.0 mm, and a surface pore ratio in the range of 6 to 90%. Since the surface pore ratio is high, the resistance to filtration is small even when the pore diameter is decreased and the filtering time can be shortened without any sacrifice in the ratio of leukocyte removal. Owing to the high surface pore ratio, the filter material of this invention obviates the necessity for such extra work as slicing or surface abrasion otherwise required in the case of polyvinyl alcohol sponge or synthetic leather of polyurethane and, as the result, the possibility of the filtrate being defiled with ground abrasive or other similar foreign matter is nil. Since the filter material has a small wall thickness, it enjoys the advantage that the priming volume can be decreased and the filter material itself can be used as stratified in a plurality of plies and the module design can be attained with a high degree of freedom.

This invention further is directed to a method for the production of a leukocyte-seizing filter material comprising preparing a raw material composition comprising at least one polymeric substance, a good solvent for the polymeric substance, and at least one pore-forming agent soluble or swellable in a poor solvent compatible with the good solvent, applying a coating of the composition on the surface of a porous substrate, leading the coated substrate into and immersing in a bath containing a poor solvent and a good solvent relative to the polymeric substance thereby effecting solidification of the coating, and thereafter allowing the pore-forming agent to be dissolved into the poor solvent and consequently effecting removal thereof from the coated substrate. It, therefore, permits easy production of a leukocyte-seizing filter material possessing such outstanding characteristic properties as described above.

Since the coagulating bath contains a good solvent, the produced filter material acquires a high surface pore ratio. Since the filter material is applied in the form of a coating on a porous substrate, it has no problem of strength in spite of the small wall thickness and avoids shrinkage even in a dry state and encounters no decline of surface pore ratio. When the porous substrate to be used is adapted so as to be usable as a prefilter, the otherwise inevitable step of incorporating a prefilter can be omitted.

BRIEF DESCRIPTION OF THE DRAWINGS

Fig. 1 is a cross section illustrating the construction of a leukocyte-seizing filter using a leukocyte-seizing filter material as one embodiment of the present invention.

Fig. 2 is a circuit diagram illustrating a blood treating circuit incorporating therein a leukocyte-seizing filter using a leukocyte-seizing filter material as one embodiment of the present invention, and

Fig. 3 is a diagram illustrating the construction of a device to be used for determining the ratio of leukocyte removal.

EXPLANATION OF THE PREFERRED EMBODIMENT

The leukocyte-seizing filter material of the present invention is characterized by being formed of a three-dimensional reticularly continuous porous material comprising at least one polymeric substance and possessing an average pore diameter in the range of 1 to 60 $\mu$m, a specific surface area in the range of 0.5 to 10 m$^2$/g, a porosity in the range of 30 to 95%, a bubble point in the range of 0.08 to 0.40 kg/cm$^2$, and a wall thickness in the range of 0.3 to 9.0 mm.

When a leukocyte suspension such as blood or concentrated red blood corpuscles is treated with a polymeric porous material whose matrix possesses a three-dimensional reticularly continuous texture and whose pore diameter, specific surface area, porosity, bubble point, and wall thickness severally fall in such specific ranges as mentioned above, the leukocytes contained in the leukocyte suspension are efficiently seized as adsorbed on the inner walls of the continuously open pores formed in the matrix of the porous material while the leukocyte suspension is flowing through complicate flow paths formed of the pores. The flow paths of the filter material are continuous open pores formed by the three-dimensional reticularly continuous texture of the porous material, namely the porous matrix. Since the flow paths of the filter material are brought into existence while the porous material is molded, the process for the production of the leukocyte-seizing filter material using the porous material is very simple and the dispersion of quality among the products obtained by the method of this invention is small. Further, since the matrix of the porous material is a continuous texture and is consequently stable, it has substantially no possibility of exuding foreign matter from the porous material or inducing the phenomenon of channeling of flow paths. Further, the polymeric porous material of the kind contemplated by this invention can be formed of such a resin as polyurethane which is relatively resistant to heat, it is capable of fully withstanding the high-pressure steam to be used for sterilization.

Now, the present invention will be described further in detail below with reference to embodiments.

In the leukocyte-seizing filter material of this invention constructed as described above, the continuous open pores are required to have an average pore diameter in the range of 1 to 60 $\mu$m, more desirably 2 to 50 $\mu$m, and most desirably 5 to 20 $\mu$m. If the pore diameter is less than 1 $\mu$m, the filter material while in use for the removal of leukocytes from the leukocyte suspension such as blood or concentrated red blood corpuscles is suffered to seize additionally red blood corpuscles contained in the leukocyte suspension and consequently entails a decrease in the ratio of red blood corpuscles recovery and clogging of the porous texture with seized red blood corpuscles present in an overwhelming number. Conversely, if the pore diameter exceeds 60 $\mu$m, there ensues the possibility that the frequency of contact of the filter material with the leukocyte suspension under treatment will be lowered and the ratio of seizure of leukocytes will be consequently decreased. The term "average pore diameter" as used in the specification of this invention refers to the magnitude to be determined by the mercury injection method.

In the leukocyte-seizing filter material of the present invention, the specific surface area is required to be in the range of 0.5 to 10 m$^2$/g, more preferably 1 to 5 m$^2$/g, and most preferably 1.5 to 3.0 m$^2$/g. If the specific surface area is less than 0.5 m$^2$/g, the amount of leukocytes to be seized is too small for the filter material to fit practical use fully. Conversely, if the specific surface area exceeds 10 m$^2$/g, there arises the possibility of the pore diameter unduly decreasing. The term "specific surface area" as used in the specification of this invention refers to the magnitude to be determined by the mercury injection method.

The porosity of the leucocyte-seizing filter material of the present invention is required to be in the range of 30 to 95%, more desirably 50 to 95%, and most desirably 60 to 95%. If the porosity is less than 30%, the treatment for the removal of leukocytes consumes too much time for the filter material to fit practical use fully. Conversely, if the porosity exceeds 95%, the filter material suffers from inferior strength. The term "porosity" as used in the specification of the present invention refers to the magnitude to be determined by the mercury injection method.

Further, in the leukocyte-seizing filter material of the present invention, the bubble point is required to be in the range of 0.08 to 0.40 kg/cm$^2$, more preferably 0.13 to 0.27 kg/cm$^2$. If the bubble point is less than 0.08 kg/cm$^2$, leakage of leukocytes occurs in the liquid recovered by the filtration. Conversely, if the bubble point exceeds 0.40 kg/cm$^2$, the possibility of the filter material being clogged unduly increases. The term "bubble point" as used in the specification of this invention is an expression widely used in the field of porous filter materials and represents the pressure with which air is forced out through the pores in a given filter material used in a thoroughly wet state.

7

Further in the leukocyte seizing filter material of this invention, the wall thickness is required to be approximately in the range of 0.3 to 9.0 mm, more preferably 0.5 to 5.0 mm, and most preferably 0.5 to 3.0 mm. If the wall thickness is less than 0.3 mm, the filter material while in use for the removal of leukocytes is incapable of seizing leukocytes in a sufficiently large amount. Conversely, if the wall thickness exceeds 9.0 mm, the speed at which the filter material effects the treatment is too slow for the filter material to fit practical use fully.

The polymeric substance of which the leukocyte-seizing filter material of this invention is formed has no particular restriction except for the sole requirement that it should withstand the heat of the high-pressure steam to be used for sterilization. Various substances are usable. Specifically, the substances which are advantageously usable herein include polyurethane, polyvinylidene fluoride, polysulfones, polyether sulfones, polyesters, poly(meth)acrylates, butadiene-acrylonitrile copolymer, polyamides, polyether-polyamide block copolymers, and ethylene-vinyl alcohol copolymers, for example. Since the leukocyte-seizing filter material of the present invention enjoys the relatively satisfactory heat-resisting property of the polymeric substance and fully withstands the conditions for sterilization with high-pressure steam, it can be used advantageously as a leukocyte-seizing filter material of the in-line type which is incorporated in the blood bag system, for example. Polyurethane may be cited as one of the desirable examples of the polymeric substance of this description.

The leukocyte-seizing filter material of this invention which possesses such characteristic properties as described above may be produced, for example, as follows. This production is specifically attained by preparing a raw material composition comprising at least one polymeric substance, a good solvent for the polymeric substance, and at least one pore-forming agent soluble or swellable in a poor solvent compatible with the good solvent, extruding the composition in a prescribed shape with an extruding device, and leading the shaped mass of the composition into and immersing in a bath formed mainly of a poor solvent relative to the polymeric substance thereby gelling the shaped mass and, at the same time, causing the pore-forming agent to be dissolved out into the poor solvent.

In this method of production, one member or a mixture of two or more members selected from the group of polymeric substances cited above is used. The amount of the polymeric substance to be used is proper in the range of 5 to 70% by weight, preferably 10 to 50% by weight, based on the total amount of the raw material composition. If the amount of the polymeric substance to be used is less than 5% by weight, there is a strong possibility that the produced porous material will not acquire sufficiently high strength. Conversely, if the amount exceeds 70% by weight, the raw material composition suffers from difficulty of handling because of undue increase of viscosity.

The good solvent for the polymeric substance is required to be capable of dissolving the polymeric substance to be used and, at the same time, compatible with the poor solvent to be used for gelling the shaped mass of the composition. It can be properly selected to suit the particular kind of the polymeric substance to be actually used. The good solvents which are usable effectively herein include dimethyl formamide, dimethyl sulfoxide, acetone, dioxane, methyl cellosolve acetate, tetrahydrofuran, ethyl alcohol, methyl alcohol, methylethyl ketone, phenol, formic acid, aromatic hydrocarbons, chlorinated hydrocarbons, and fluorinated alcohols, for example.

Though the pore-forming agent is variable with the kind of polymeric substance and the kind of solvent to be actually used, it is required to be soluble or swellable in a poor solvent compatible with the good solvent for the polymeric substance mentioned above. For the sake of the ease of handling, it is desired to be a water-soluble compound like a water-soluble polymer. Where dimethyl formamide, dimethyl sulfoxide, acetone, or ethyl acetate which is soluble in water or alcohol which is generally used as a non-solvent is selected as a good solvent, for example, the pore-forming agents which are usable herein include polyvinyl alcohol, polyvinyl pyrrolidone, methyl cellulose, polyethers, polysaccharides, polyacrylic acid and salts thereof, and polyacrylamide, for example. The amount of the pore-forming agent to be added is required to be approximately in the range of 1 to 60% by weight, preferably 5 to 50% by weight, based on the total amount of the raw material composition. If the amount of the pore-forming agent is less than 1% by weight, there is a great possibility of the produced porous material failing to acquire such a three-dimensional reticularly continuous texture or the matrix of the porous material failing to form therein continuous open pores. Conversely, if the amount exceeds 60% by weight, the raw material composition suffers from difficulty of handling because of undue rise of viscosity.

The raw material composition, when necessary, may incorporate therein additionally a pore diameter-adjusting agent in an amount of not more than 50% by weight, preferably in the range of 3 to 20% by weight, based on the total amount of the raw material composition. This pore diameter-adjusting agent is required to be soluble in the poor solvent for the polymeric substance and insoluble in the good solvent and either compatible with or insoluble and yet uniformly miscible with the pore-forming agent. The pore diameter-adjusting agents which are advantageously usable herein include alginic acid, carboxymethyl cellulose, polyacrylates, various species of starch, dextrin, and chlorides, sulfates, and other similar inorganic salts of sodium, potassium, calcium, strontium, and aluminum.

The preparation of the raw material composition comprising the components described above is carried out at a temperature not exceeding the boiling point of the solvent, preferably at a temperature approximately in the range of 10° to 80°C. The components are thoroughly stirred until there is formed a homogeneous dispersion.

The raw material composition prepared as described above is then extruded in a prescribed shape by the use of an extruding device provided with a suitable die such as, for example, a T die which has a flat slit formed in the leading end thereof. The extruded mass of the composition is led into a bath formed mainly of a poor solvent for the polymeric substance. In consequence of the immersion in this bath, the shaped mass of the composition is gelled and, at the same time, the pore-forming agent is dissolved out of the shaped mass into the poor solvent.

The poor solvent for the polymeric substance is only required to exhibit compatibility to the good solvent to be used. Generally, water or an alcohol or a mixture thereof is used as mentioned above. The bath to be used for the gelation mentioned above, when necessary, may incorporate therein a good solvent for the polymeric substance in addition to the poor solvent mentioned above. For the gelation to be attained advantageously, the bath is desired to be retained at a temperature approximately in the range of 0° to 70°C, preferably 15° to 60°C. Where this immersion in the bath is not enough to attain thorough removal of the pore-forming agent from the shaped mass of composition, this step of immersion may be followed by an additional step of washing with a poor solvent as occasion demands.

The leukocyte-seizing filter material of this invention which possesses such characteristic properties as described above is otherwise produced by preparing a raw material composition comprising at least one polymeric substance, a good solvent for the polymeric substance, and at least one pore-forming agent soluble or swellable in a poor solvent compatible with the good solvent, applying a coating of the composition on the surface of a substrate, and leading the coated substrate into and immersing in a bath formed mainly of a poor solvent relative to the polymeric substance thereby gelling the coating and, at the same time, causing the pore-forming agent to be dissolved out of the coating into the poor solvent.

No particular method is used for the application of the composition in the form of a coating on the surface of a substrate. Any of the well-known methods such as, for example, the doctor knife method, roller coating method, dipping method, and spin coating method may be adopted for the application.

In the second method of production just described the details of process involved are substantially similar to those of the first method except for the manner in which the composition is molded. The description of these details, therefore, are omitted here.

The leukocyte-seizing filter material of the present invention is characterized by having formed on the surface of a porous substrate a three-dimensional reticularly continuous porous material comprising at least one polymeric substance and possessing an average pore diameter in the range of 1 to 60 $\mu$m, a specific surface area in the range of 0.5 to 10 m$^2$/g, a porosity in the range of 30 to 95%, a bubble point in the range of 0.08 to 0.30 kg/cm$^2$, a wall thickness in the range of 0.1 to 9.0 mm, and a surface pore ratio in the range of 6 to 90%.

In the three-dimensional reticularly continuous porous layer of the leukocyte-seizing filter material of the present invention which is constructed as described above, the average pore diameter of the continuous open pores is required to be in the range of 1 to 60 $\mu$m, more preferably 2 to 50 $\mu$m, and most preferably 5 to 20 $\mu$m. The reason for this specific range of the average pore diameter is as already described above.

In the continuous porous layer of the leukocyte-seizing filter material of the present invention, the bubble point is desired to be in the range of 0.08 to 0.30 kg/cm$^2$, preferably 0.13 to 0.27 kg/cm$^2$. If the bubble point is less than 0.08 kg/cm$^2$, there occurs leakage of leukocytes in the liquid recovered by the filtration. Conversely, if the bubble point exceeds 0.30 kg/cm$^2$, there ensues a great possibility of the filter material being clogged. The term "bubble point" as used herein is an expression widely used in the field of porous filter materials and refers to the pressure with which air is forced out through the pores in the filter material in a thoroughly wet state.

Further, the wall thickness of the three-dimensional reticularly continuous porous layer of the filter material of this invention is required to be approximately in the range of 0.1 to 9.0 mm, more desirably 0.3 to 5.0 mm, and most desirably 0.5 to 3.0 mm. If the wall thickness of this porous material is less than 0.1 mm, the filter material while in use for the removal of leukocytes is incapable of seizing leukocytes in a fully satisfactory amount. Conversely, if the wall thickness exceeds 9.0 mm, the filter material is deficient in practicability because of unduly slow speed of treatment.

9

Further, in the continuous porous layer of the leukocyte-seizing filter material of the present invention, the surface pore ratio is required to be in the range of 6 to 90%, more desirably 10 to 85 %, and most desirably 13 to 80 %.

The polymeric substances which are available for the formation of the three-dimensional reticularly continuous porous layer in the leukocyte-seizing filter material of the present invention are as already described above.

As the material for the porous substrate to be used in the present invention, any material can be adopted on the condition that (1) it should offer virtually no resistance to passage of blood, (2) it should be harmless to blood (incapable of causing hemolysis or activation of platelets, for example), (3) it should allow adhesion thereto of the three-dimensional reticularly continuous porous layer by virtue of physical anchoring and allow no ready separation thereof, (4) it should possess rigidity enough to withstand the force with which the porous layer shrinks in the course of drying, and (5) it should avoid being readily affected by the solvent used in the raw material for the porous layer, the solvent in the coagulating bath, and water. Preferably, the selected material is required to be sterilizable by the use of an autoclave. The materials which fulfil these conditions include polyesters, polyamides, polyacrylate, and polyolefins, for example. The forms in which the porous substrate contemplated by the present invention is usable herein include non-woven fabric, woven fabric, knit fabric, meshes, and foam, for example. Though the thickness of the porous substrate is not specifically limited, it is generally desired to be in the range of 0.05 to 1 mm, preferably 0.1 to 0.5 mm. When the porous substrate is used, so long as the porous substrate has no adverse effect of any sort upon the operation of the filter material for the seizure of leukocytes, it is not required to be separated from the porous material which has been formed by gelling the raw material composition and causing the pore-forming agent to be dissolved out of the molded mass of the composition into the poor solvent in accordance with the present invention. Thus, the porous substrate may be allowed to function as a support for the porous material.

The leukocyte-seizing filter material of the present invention which possesses such characteristic properties as described above is produced, for example, as follows. This production is effected specifically by preparing a raw material composition comprising at least one polymeric substance, a good solvent for the polymeric substance, and at least one pore-forming agent soluble or swellable in a poor solvent compatible with the good solvent, applying a coating of the composition on the surface of a porous substrate, leading the coated substrate into and immersing in a bath containing a poor solvent and a good solvent for the polymeric substance thereby solidifying the applied coating, and thereafter causing the pore-forming agent to be dissolved out into the poor solvent.

In this method of production, one member or a mixture of two or more members selected from the group of polymeric substances mentioned above is used.

The good solvents for the polymeric substance and the pore-forming agent to be used in the method are as already described above.

Optionally, the raw material composition may incorporate therein a pore diameter-adjusting agent in an amount of not more than 50% by weight, preferably in the range of 3 to 20% by weight, based on the total amount of the raw material composition. The amount of this pore diameter-adjusting agent and the action thereof are as already described above.

The preparation of the raw material composition comprising the components described above is carried out at a temperature not exceeding the boiling point of the solvent, preferably at a temperature approximately in the range of 10° to 80°C. The components are thoroughly stirred until there is formed a homogeneous dispersion.

The raw material composition prepared as described above is then extruded in a prescribed shape by the use of an extruding device provided with a suitable die such as, for example, a T die which has a flat slit formed in the leading end thereof, applied in the form of a coating on the surface of a substrate, and led into a bath formed mainly of a poor solvent and a good solvent for the polymeric substance. In consequence of the immersion in this bath, the shaped mass of the composition is gelled and, at the same time, the pore-forming agent is dissolved out of the shaped mass into the poor solvent.

The poor solvent for the polymeric substance is only required to exhibit compatibility to the good solvent to be used. Generally, water or an alcohol or a mixture thereof is used as mentioned above. The bath to be used for the gelation mentioned above, when necessary, may incorporate therein a good solvent for the polymeric substance in addition to the poor solvent mentioned above. For the gelation to be attained advantageously, the bath is desired to be retained at a temperature approximately in the range of 0° to 70°C, preferably 15° to 60°C. Where this immersion in the bath is not enough to attain thorough removal of the pore-forming agent from the shaped mass of composition, this step of immersion may be followed by an additional step of washing with a poor solvent as occasion demands.

No particular method is used for the application of the composition in the form of a coating on the surface of a substrate. Any of the well-known methods such as, for example, the doctor knife method, roller coating method, dipping method, and spin coating method may be adopted for the application.

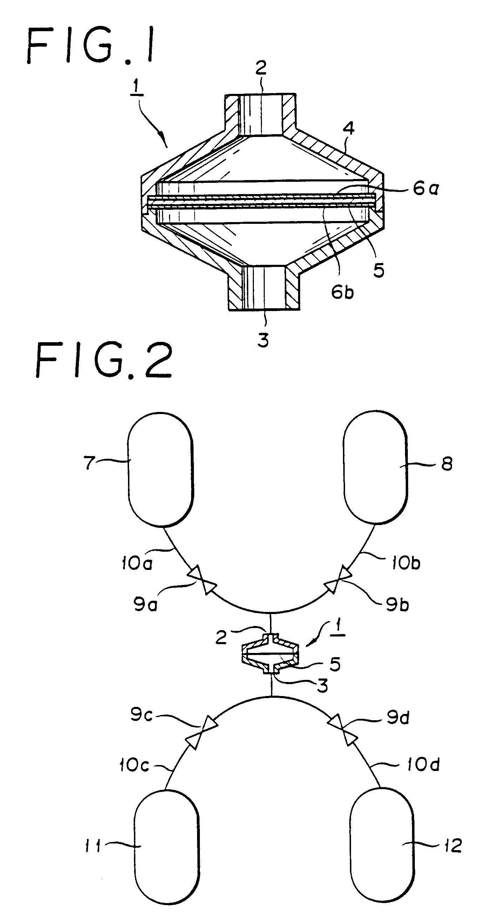

Fig. 1 is a cross section of a leukocyte-seizing filter using a leukocyte-seizing filter material as one embodiment of this invention. In the present embodiment, a leukocyte-seizing filter 1 comprises a housing 4 provided with a blood inlet 2 and a blood outlet 3 and a leukocyte-seizing filter material 5 of the construction described above disposed across the empty space inside the housing 4. In the leukocyte-seizing filter 1, for fast retention of the leukocyte-seizing filter material 5 inside the housing 4, liquid-pervious supporting members 6a, 6b may be disposed one each on the opposite surfaces of the leukocyte-seizing filter material 5 in such a manner as to nip the leukocyte-seizing material 5. This leukocyte-seizing filter 1 is actually used as incorporated in a circuit as illustrated in Fig. 2, for example. In the circuit illustrated in Fig. 2, a blood bag 7 containing the blood to be treated and a physiological saline solution bag 8 containing physiological saline solution are each positioned above the leukocyte-seizing filter 1 and are each allowed to communicate with the blood inlet 2 of the leukocyte-seizing filter 1 through liquid-guiding tubes 10a, 10b provided respectively with clamps 9a, 9b. A physiological saline solution recovery bag 11 and a blood recovery bag 12 for recovering the treated blood are disposed below the leukocyte-seizing filter 1 and are allowed to communicate with the blood outlet 3 of the leukocyte-seizing filter 1 through liquid-guiding tubes 10c, 10d provided respectively with clamps 9c, 9d. The operation of the circuit for the separation of leukocytes is initiated by first opening the clamps 9b, 9c and, with the clamps 9a, 9d kept in a closed state, allowing the physiological saline solution to flow from the physiological saline solution bag 8 to the leukocyte-seizing filter 1, and priming the interior of the leukocyte-seizing filter 1. The physiological saline solution which has been thus used for the priming is recovered in the physiological saline solution recovery bag 11. After the priming has been carried out, the clamps 9a, 9d are opened and the clamps 9b, 9c are closed and the blood is allowed to flow from the blood bag 7 into the leukocyte-seizing filter 1. While the blood is passing through the leukocyte-seizing filter material 5 constructed as described above inside the leukocyte-seizing filter 1, the leukocytic component of the blood is seized by adsorption by the leukocyte-seizing filter material 5 and the blood is converted into the form deprived of leukocytes. The blood which is now divested with the leukocytic component is recovered in the blood recovery bag 12 with which the leukocyte-seizing filter 1 is connected. When the flow of the blood from the blood bag 7 is completed, the blood remaining in the leukocyte-seizing filter 1 is expelled out of the leukocyte-seizing filter 1 and recovered in the blood recovery bag 12 by opening the clamp 9b and advancing the flow of physiological saline solution into the leukocyte-seizing filter 1. At the time that the recovery of the blood is practically completed, the operation of the circuit for the separation of leukocytes is terminated by closing the clamp 9d, opening the clamp 9c and recovering the physiological saline solution used for the recovery of the blood into the physiological saline solution recovery bag 11.

Now, the present invention will be described more specifically below with reference to working examples.

Examples 1 to 5 and Controls 1 and 2

In dimethyl formamide (DMF), polyurethane (produced by Nippon Elastran K.K. and marketed under trademark designation of "Paraplen P395R NAT") was dissolved in a concentration of 20 w/v% at 50°C. The resultant hot solution was cooled to room temperature and methyl cellulose as a pore-forming agent was added to the cooled solution in a ratio indicated in Table 1. Then, by thoroughly stirring the mixture at 30°C, a dope was prepared.

Then, the dope prepared as described above was placed in an extruding device provided with a T die, discharged through a die slit at a temperature of 50°C into a water bath at a temperature of 30°C, and consequently allowed to gel. Then, the product of gelation was washed with water at 60°C to expel the pore-forming agent therefrom and give rise to a porous material.

The porous material thus obtained was subjected to displacement with alcohol, then dried, and sterilized with high-pressure steam at 121°C for 20 minutes, and tested for properties as described hereinbelow. The results are shown in Table 1 and Table 2.

Examples 6 to 8 and Controls 3 and 4

In dimethyl formamide (DMF), polyurethane (produced by Nippon Elastran K.K. and marketed under trademark designation of "Paraplen P395R NAT") was dissolved in a concentration of 20 w/v% at 50°C. The resultant hot solution was cooled to room temperature and polyvinyl alcohol was added thereto as a pore-forming agent in a ratio indicated in Table 1. Then, by thoroughly stirring the mixture at 30°C, a dope was prepared.

The dope prepared as described above was applied in the form of a coating on the surface of a glass substrate by the use of a coater at room temperature. The applied coating of the dope as superposed on the glass substrate was immersed in a water bath at 30°C to gel the dope. Then, the product of the gelation was washed with water at 60°C to expel the pore-forming agent therefrom and give rise to a porous material.

The porous material thus obtained was subjected to displacement with alcohol, then dried, and further sterilized with high-pressure steam at 121°C for 20 minutes, and tested in the same manner as in Example 1. The results are shown in Table 1 and Table 2.

Control 5

A commercially available polyvinyl formal sponge was used as a filter material and tested in the same manner as in Example 1. The results are shown in Table 1 and Table 2.

Control 6

A commercially available polyurethane foam was used as a filter material and tested in the same manner as in Example 1. The results are shown in Table 1 and Table 2.

TEST

(1) Average pore diameter

This property was determined by the use of a mercury injection meter (produced by Carloelva Co. and marketed under trademark designation of "Macropore Unit 120 & Porosimeter 2000).

(2) Specific surface area

This property was determined by the use of a mercury injection meter (produced by Carloelva Co. and marketed under trademark designation of "Macropore Unit 120 & Porosimeter 2000).

(3) Porosity

This property was determined by the use of a mercury injection meter (produced by Carloelva Co. and marketed under trademark designation of "Macropore Unit 120 & Porosimeter 2000).

(4) Bubble point

This property was determined by completely wetting with water one surface of a given porous material immediately prior to the determination, gradually increasing the air pressure applied to the other surface of the porous material, and measuring the pressure, i.e. bubble point, at which steady and continuous flow of fine bubbles through the filter is detected.

(5) Wall thickness

This property was determined by the use of a micrometer.

(6) Ratio of removal of leukocytes

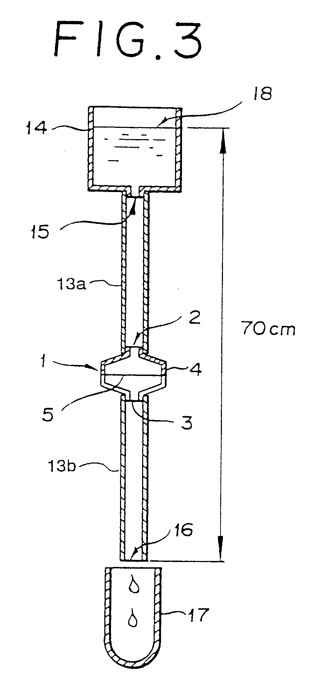

This property was determined with an apparatus which was constructed by punching out a porous material 4.7 cm in diameter, fixing the disk of porous material within a housing 4 provided with a blood inlet 2 and a blood outlet 3 as illustrated in Fig. 3, connecting a blood outlet 15 of a blood container 14 through the medium of a polyvinyl chloride tube 13a to the blood inlet 2 of the filter 1, connecting to the blood outlet 3 of the filter 1 a polyvinyl chloride tube 13b having a nearly equal length to the tube 13a, and disposing a blood recovery container 17 below the open terminal 16 of the tube 13b, with a distance of 70 cm interposed between the liquid level 18 of the blood placed in the blood container 14 and the open terminal

12

16 of the tube 13b. The determination of this property was effected by allowing 50 ml of blood to flow owing to the difference in level, calculating the numbers of leukocytes in the blood before and after the treatment with an automatic blood cell measuring device (produced by Ortho-Instrument Corp. and marketed under product code of "ELT-8"), and performing calculation in accordance with the following formula.

Ratio of removal of leukocytes (%) = [1 - (number of leukocytes after filtration)/(number of leukocytes before filtration)] x 100

Table 1

| Example | Pore-forming agent | Weight ratio of polyurethane (polyurethane = 100) | Average pore diameter (μm) | Specific surface area (m²/g) | Porosity (%) | Bubble point (kg/cm²) | Wall thickness (mm) |
|---|---|---|---|---|---|---|---|
| Example 1 | Methyl cellulose | 50 | 1.3 | 0.56 | 39 | 0.32 | 1.5 |
| Example 2 | Methyl cellulose | 100 | 4.0 | 3.51 | 56 | 0.24 | 1.5 |
| Example 3 | Methyl cellulose | 200 | 8.6 | 2.58 | 71 | 0.19 | 1.5 |
| Example 4 | Methyl cellulose | 300 | 29.9 | 1.32 | 86 | 0.10 | 1.5 |
| Example 5 | Methyl cellulose | 400 | 54.3 | 1.54 | 91 | 0.08 | 1.5 |
| Control 1 | Methyl cellulose | 30 | 0.79 | 0.24 | 27 | 0.45 | 1.5 |
| Control 2 | Methyl cellulose | 500 | 62.2 | 1.28 | 80 | 0.08 | 1.5 |
| Example 6 | Polyvinyl alcohol | 100 | 3.4 | 2.46 | 46 | 0.24 | 1.0 |
| Example 7 | Polyvinyl alcohol | 200 | 7.8 | 3.16 | 72 | 0.22 | 1.0 |
| Example 8 | Polyvinyl alcohol | 300 | 9.3 | 2.72 | 81 | 0.17 | 1.0 |
| Control 3 | Polyvinyl alcohol | 30 | 0.64 | 0.37 | 36 | 0.48 | 1.0 |
| Control 4 | Polyvinyl alcohol | 500 | 63.5 | 1.45 | 82 | 0.10 | 1.0 |
| Control 5 | - | - | 8.0 | 2.25 | 86 | 0.15 | 1.0 |
| Control 6 | - | - | 19.0 | 1.05 | 96 | 0.05 | 10.0 |

Table 2

| Example | Removal ratio of leukocytes (%) | Filtering time (min) | Clogging |
|---|---|---|---|
| Example 1 | 100 | 110 | Little yes |
| Example 2 | 100 | 63 | Slightly yes |
| Example 3 | 98 | 8 | No |
| Example 4 | 89 | 4 | No |
| Example 5 | 72 | 2 | No |
| Control 1 | No measurable | No measurable | Yes (stopped in the way) |
| Control 2 | 40 | 1 | No |
| Example 6 | 100 | 55 | Slightly yes |
| Example 7 | 97 | 8 | Little yes |
| Example 8 | 90 | 7 | Yes |
| Control 3 | No measurable | No measurable | No |
| Control 4 | 44 | 1 | No |
| Control 5 | 99 | 8 | No |
| Control 6 | 48 | 3 | No |

Control 7

The same commercially available polyvinyl formal sponge as used in Control 5 was subjected to sterilization with high-pressure steam at 121°C for 20 minutes. An attempt at subjecting the sterilized sponge to the test in the same manner as described above failed because it was seriously deformed by the heat of the sterilization.

Example 9 and Control 8

Porous materials produced by the procedures of Example 3 and Control 6 were tested for determination of the ratio of removal of leukocytes in the same manner as described above. Then, 50 ml of physiological saline solution containing blood plasma was caused to flow at a flow rate of 5 ml/min. through each of the porous materials, the porous material kept shaken, to recover leukocytic cells seized in the porous material.

As the result, the porous material of the procedure of Control 6 (sample of Control 8) recovered the seized leukocytes in a high ratio of 50%, whereas the porous material of the procedure of Example 3 (sample of Example 9) recovered the seized leukocytes only in a low ratio of several %.

These results suggest that the leukocyte-seizing filter according with the present invention provides more infallible seizure of leukocytic cells owing to the three-dimensional reticular structure.

Examples 10 to 14 and Controls 9 and 10

In N-methyl pyrrolidone (produced by Mitsubishi and marketed under product code of "NMP"), polyurethane (produced by Nippon Miractran K.K. and marketed under trademark designation of "Miractran E395 NAT") was dissolved in a concentration of 15 w/v% at 50°C and methyl cellulose as a pore-forming agent was added thereto in a ratio indicated in Table 3 and calcium chloride dihydrate as a pore diameter-adjusting agent was further added thereto in one tenth of the amount of methyl cellulose. With a twin-axis planatary mixer (produced by Inoue Seisakusho K.K.), the resultant mixture was kneaded at 50°C for 1 hour for vacuum deaeration, to prepare a dope.

The dope prepared as described above was applied in the form of a coating on a polyester non-woven fabric (0.15 mm in thickness) with a coater at a temperature of 50°C. The applied coating of the dope and the non-woven fabric were jointly immersed in an aqueous 50% NMP solution at 50°C for 1 hour to solidify the dope. Thereafter, the product of the coagulation was washed in running water to expel the pore-forming agent out of the coating and give rise to a porous material.

The porous material was dried in an oven at 60°C, sterilized with high-pressure steam at 121°C for 20 minutes, and put to the following test. The results are shown in Table 3 and Table 4.

Examples 15 to 18 and Control 11

Porous materials were produced by following the procedures of Examples 10 to 14, excepting the amount of methyl cellulose added was changed to 150 phr relative to the amount of polyurethane and the NMP concentration in the coagulating bath was changed as indicated in Table 3 and Table 4. These porous materials were subjected to the same test as mentioned above. The results are shown in Table 4.

Control 12

The same polyester non-woven fabrics (produced by Nippon By lean K.K. and marketed under product code of "JH-104N") as used in Examples 10 to 18 and Controls 9 to 11 were tested in the same manner as described above. The results are shown in Table 4.

The tests for average pore diameter, specific surface area, porosity, bubble point, wall thickness, and ratio of removal of leukocytes were conducted as described above. The surface pore ratio was determined by the following method.

(7) Surface pore ratio

This property was determined by observing the surface of a given filter material under a scanning electron microscope (produced by Japan Electron Optics Laboratory Co., Ltd. and marketed under product code of "JSM-840") at 500 magnifications, supplying the image on-line to an image analyzer (produced by Tokyo Shibaura Electric Co., Ltd. and marketed under trademark designation of "TOSPIX-U"), and finding the ratio of the surface area of the pores to the surface area of the image in percentage.

Table 3

| | Weight ratio of polyurethane (polyurethane = 100) | NMP concentration in coagulating bath (V/W %) | Average pore diameter (µm) | Specific surface area (m²/g) | Porosity (%) | Buble point (kg/cm²) | Wall thickness (mm) | Surface pore ration (%) |
|---|---|---|---|---|---|---|---|---|
| Control 9 | 30 | 50 | 0.81 | 0.27 | 27 | 0.38 | 0.3 | 7.4 |
| Example 10 | 50 | 50 | 2.3 | 0.58 | 41 | 0.19 | 0.3 | 28 |
| Example 11 | 100 | 50 | 7.8 | 1.94 | 53 | 0.13 | 0.3 | 38 |
| Example 12 | 125 | 50 | 9.7 | 2.41 | 60 | 0.13 | 0.3 | 46 |
| Example 13 | 150 | 50 | 12.8 | 3.13 | 69 | 0.12 | 0.3 | 47 |
| Example 14 | 300 | 50 | 31.2 | 2.65 | 77 | 0.10 | 0.3 | 53 |
| Control 10 | 500 | 50 | 62.2 | 1.26 | 80 | 0.07 | 0.3 | 74 |
| Control 11 | 125 | 0 | 8.3 | 2.46 | 42 | 0.46 | 0.3 | 5.0 |
| Example 15 | 125 | 20 | 8.3 | 2.41 | 44 | 0.26 | 0.3 | 17 |
| Example 16 | 125 | 40 | 8.8 | 2.47 | 58 | 0.19 | 0.3 | 57 |
| Example 17 | 125 | 60 | 10.3 | 2.83 | 61 | 0.13 | 0.3 | 60 |
| Example 18 | 125 | 80 | 19.2 | 2.11 | 63 | 0.09 | 0.3 | 65 |

Table 4

| Example | Removal ratio of leukocytes (%) | Filtering time (min) | Clogging |
|---|---|---|---|
| Control 9 | - | - | Yes (stopped in the way) |
| Example 10 | 100 | 22′13″ | No |
| Example 11 | 100 | 4′07″ | No |
| Example 12 | 99 | 3′28″ | No |
| Example 13 | 96 | 3′03″ | No |
| Example 14 | 83 | 2′04″ | No |
| Control 10 | 41 | 1′57″ | No |
| Control 11 | - | - | Yes (stopped in the way) |
| Example 15 | 100 | 17′43″ | No |
| Example 16 | 100 | 10′37″ | No |
| Example 17 | 98 | 4′08″ | No |
| Example 18 | 77 | 1′59″ | No |
| Control 12 | 15.5 | 16′31″ | No |

**Claims**

1. A leukocyte-seizing filter material formed of a three-dimensional reticularly continuous porous material comprising at least one polymeric substance, wherein said filter material possesses an average pore diameter in the range of 1 to 60 $\mu$m, a specific surface area in the range of 0.5 to 10m$^2$/g, a porosity in the range of 30 to 95%, a bubble point in the range of 0.08 to 0.40 kg/cm$^2$, and a wall thickness in the range of 0.3 to 9.0 mm, and which is sterilizable with high-pressure steam.

2. A filter material according to claim 1, wherein said polymeric substance is one member selected from the group consisting of polyurethane, polyvinylidene fluoride, polysulfones, polyether sulfones, polyesters, poly(meth)acrylates, butadiene-acrylonitrile copolymers, polyamides, polyether polyamide block copolymers, and ethylene-vinyl alcohol copolymers.

3. A filter material according to claim 1, wherein said polymeric substance is polyurethane.

4. A filter material according to claim 1, wherein said average pore diameter is in the range of 2 to 50 $\mu$m, said specific surface area in the range of 1 to 5 m$^2$/g, said porosity in the range of 50 to 95%, said bubble point in the range of 0.13 to 0.27 kg/cm$^2$, and said wall thickness in the range of 0.5 to 5.0 mm.

5. A filter material according to claim 1 wherein a layer of the three-dimensional reticularly continuous porous material is formed on the surface of a porous substrate and wherein the filter material has a bubble point in the range of 0.08 to 0.30 kg/cm$^2$ and a surface pore ratio in the range of 6 to 90%.

6. A filter material according to claim 5, wherein said polymeric substance is one member selected from the group consisting of polyurethane, polyvinylidene fluoride, polysulfones, polyether sulfones, polyesters, poly(meth)acrylates, butadiene-acrylonitrile copolymers, polyamides, polyether polyamide block copolymers, and ethylene-vinyl alcohol copolymers.

7. A filter material according to claim 5, wherein said polymeric substance is polyurethane.

8. A filter material according to claim 5, wherein said average pore diameter is in the range of 2 to 50 $\mu$m, said specific surface area in the range of 1 to 5 m$^2$/g, said bubble point in the range of 0.13 to 0.27 kg/cm$^2$, said wall thickness in the range of 0.3 to 5.0 mm, and said surface pore ratio in the range of 10

17

to 85%.

9. A filter material according to claim 5, wherein said porous substrate is non-woven fabric.

10. A method for the production of a leukocyte-seizing filter material as defined in claim 1 which comprises preparing a raw material composition comprising at least one polymeric substance, a good solvent for said polymeric substance, and at least one pore-forming agent soluble or swellable in a poor solvent relative to said polymeric substance and compatible with said good solvent, extrusion moulding said raw material composition in a prescribed shape with an extruding device, and then leading the extrusion moulded composition into and immersing in a bath formed mainly of said poor solvent thereby gelling said extrusion moulded composition and, at the same time, allowing said pore-forming agent to be dissolved into said poor solvent and consequently effecting removal thereof from said shaped composition.

11. A method according to claim 10 which further comprises applying a coating of said extrusion-moulded composition on the surface of a substrate, and leading the coated substrate into and immersing in said bath formed mainly of said poor solvent thereby gelling or effecting solidification of the applied coating and, at the same time, allowing said pore-forming agent to be dissolved into said poor solvent and consequently effecting removal thereof from said coated substrate.

12. A method according to claim 10 or claim 11, wherein said polymeric substance is one member selected from the group consisting of polyurethane, polyvinylidene fluoride, polysulfones, polyether sulfones, polyesters, poly(meth)acrylates, butadiene-acrylonitrile copolymers, polyamides, polyether polyamide block copolymers, and ethylene-vinyl alcohol copolymers.

13. A method according to claim 10 or claim 11, wherein said polymeric substance is polyurethane.

14. A method according to claim 12, wherein said pore-forming agent is a water-soluble compound.

15. A method according to claim 12, werein said pore-forming agent is a water-soluble polymer.

16. A method according to claim 14, wherein said pore-forming agent is one member selected from the group consisting of a polyvinyl alcohol, polyvinyl pyrrolidone, methyl cellulose, polyethers, polysaccharides, polyacrylic acid and salts thereof, and polyacrylamides.

17. A method according to claim 11, wherein said substrate is porous, wherein said immersion bath further contains a good solvent relative to the polymeric substance and wherein said immersed coated substrate solidifies prior to the dissolution of the pore-forming agent in the poor solvent.

18. A method according to claim 17, wherein said polymeric substance is one member selected from the group consisting of polyurethane, polyvinylidene fluoride polysulfones, polyether sulfones, polyesters, poly(meth)acrylates, butadiene-acrylonitrile copolymers, polyamides, polyether polyamide block copolymers, and ethylene-vinyl alcohol copolymers.

19. A method according to claim 17, wherein said polymeric substance is polyurethane.

20. A method according to claim 17, wherein said pore-forming agent is a water-soluble compound.

21. A method according to claim 20, wherein said pore-forming agent is a water-soluble polymer.

22. A method according to claim 20, wherein said pore-forming agent is one member selected from the group consisting of polyvinyl alcohol, polyvinyl pyrrolidone, methyl cellulose, polyethers, polysaccharides, polyacrylic acid and salts thereof, and polyacrylamides.

23. A method according to claim 17, wherein said porous substrate is non-woven fabric.

## EP 0 408 462 B1

**Patentansprüche**

1. Filtermaterial zum Abscheiden von Leukocyten, gebildet aus einem dreidimensionalen, netzartig fortlaufenden, porösen Material, umfassend mindestens eine polymere Substanz, wobei das Filtermaterial einen durchschnittlichen Porendurchmesser im Bereich von 1 bis 60 $\mu$m, eine spezifische Oberfläche im Bereich von 0,5 bis 10 m$^2$/g, eine Porosität im Bereich von 30 bis 95 %, einen Blasenpunkt im Bereich von 0,08 bis 0,40 kg/cm$^2$ und eine Wanddicke im Bereich von 0,3 bis 9,0 mm aufweist und mit Hochdruckdampf sterilisierbar ist.

2. Filtermaterial nach Anspruch 1, wobei die polymere Substanz aus einer Komponente, ausgewählt aus der Gruppe Polyurethan, Polyvinylidenfluorid, Polysulfone, Polyethersulfone, Polyester, Poly(meth)-acrylate, Butadien-Acrylnitril-Mischpolymere, Polyamide, Polyether/Polyamid-Blockmischpolymere und Ethylen-Vinylalkohol-Mischpolymere, besteht.

3. Filtermaterial nach Anspruch 1, wobei die polymere Substanz aus Polyurethan besteht.

4. Filtermaterial nach Anspruch 1, wobei der durchschnittliche Porendurchmesser im Bereich von 2 bis 50 $\mu$m, die spezifische Oberfläche im Bereich von 1 bis 5 m$^2$/g, die Porosität im Bereich von 50 bis 95%, der Blasenpunkt im Bereich von 0,13 bis 0,27 kg/cm$^2$ und die Wanddicke im Bereich von 0,5 bis 5,0 mm liegen.

5. Filtermaterial nach Anspruch 1, wobei auf der Oberfläche eines porösen Substrats eine Schicht des dreidimensionalen, netzartig fortlaufenden, porösen Material gebildet ist und wobei das Filtermaterial einen Blasenpunkt im Bereich von 0,08 bis 0,30 kg/cm$^2$ und ein Oberflächen/Poren-Verhältnis im Bereich von 6 bis 90% aufweist.

6. Filtermaterial nach Anspruch 5, wobei die polymere Substanz aus einer Komponente, ausgewählt aus der Gruppe Polyurethan, Polyvinylidenfluorid, Polysulfone, Polyethersulfone, Polyester, Poly(meth)-acrylate, Butadien-Acrylnitril-Mischpolymere, Polyamide, Polyether/Polyamid-Blockmischpolymere und Ethylen-Vinylalkohol-Mischpolymere, besteht.

7. Filtermaterial nach Anspruch 5, wobei die polymere Substanz aus Polyurethan besteht.

8. Filtermaterial nach Anspruch 5, wobei der durchschnittliche Porendurchmesser im Bereich von 2 bis 50 $\mu$m, die spezifische Oberfläche im Bereich von 1 bis 5 m$^2$/g, der Blasenpunkt im Bereich von 0,13 bis 0,27 kg/cm$^2$, die Wanddicke im Bereich von 0,3 bis 5,0 mm und das Oberflächen/Poren-Verhältnis im Bereich von 10 bis 85% liegen.

9. Filtermaterial nach Anspruch 5, wobei das poröse Substrat aus einem Vlies besteht.

10. Verfahren zur Herstellung eines Filtermaterials zum Abscheiden von Leukocyten nach Anspruch 1 durch Zubereiten einer Rohmaterialmasse, umfassend mindestens eine polymere Substanz, ein gutes Lösungsmittel für die polymere Substanz und mindestens einen in einem mit dem guten Lösungsmittel verträglichen, für die polymere Substanz schlechten Lösungsmittel löslichen oder quellbaren Porenbildner, Ausformen der Rohmaterialmasse zu einer gegebenen Form durch Strangpressen und anschließendes Hinführen der durch Strangpressen ausgeformten Masse zu einem und Eintauchen in ein Bad aus hauptsächlich dem schlechten Lösungsmittel zum Gelieren der durch Strangpressen ausgeformten Masse und, gleichzeitig, zum In-Lösunggehen-Lassen des Porenbildners in dem schlechten Lösungsmittel unter Entfernung desselben aus der ausgeformten Masse.

11. Verfahren nach Anspruch 10, weiterhin umfassend die Applikation eines Überzugs aus der durch Strangpressen ausgeformten Masse auf die Oberfläche eines Substrats und Hinführen sowie Eintauchen des beschichteten Substrats zu dem bzw. in das hauptsächlich aus dem schlechten Lösungsmittel bestehende(n) Bad zur Gelierung oder Verfestigung des applizierten Überzugs und, gleichzeitig, zum In-Lösunggehen-Lassen des Porenbildners in dem schlechten Lösungsmittel unter Entfernung desselben aus dem beschichteten Substrat.

19

**12.** Verfahren nach Anspruch 10 oder 11, wobei die polymere Substanz aus einer Komponente, ausgewählt aus der Gruppe Polyurethan, Polyvinylidenfluorid, Polysulfone, Polyethersulfone, Polyester, Poly(meth)-acrylate, Butadien-Acrylnitril-Mischpolymere, Polyamide, Polyether/Polyamid-Blockmischpolymere und Ethylen-Vinylalkohol-Mischpolymere, besteht.

**13.** Verfahren nach Anspruch 10 oder 11, wobei die polymere Substanz aus Polyurethan besteht.

**14.** Verfahren nach Anspruch 12, wobei der Porenbildner aus einer wasserlöslichen Verbindung besteht.

**15.** Verfahren nach Anspruch 12, wobei der Porenbildner aus einem wasserlöslichen Polymer besteht.

**16.** Verfahren nach Anspruch 14, wobei der Porenbildner aus einer Komponente, ausgewählt aus der Gruppe Polyvinylalkohol, Polyvinylpyrrolidon, Methylcellulose, Polyether, Polysaccharide, Polyacrylsäure und deren Salze und Polyacrylamide, besteht.

**17.** Verfahren nach Anspruch 11, wobei das Substrat porös ist, wobei das Tauchbad zusätzlich ein für die polymere Substanz gutes Lösungsmittel enthält und wobei das eingetauchte beschichtete Substrat vor dem In-Lösunggehen des Porenbildners in dem schlechten Lösungsmittel fest wird.

**18.** Verfahren nach Anspruch 17, wobei die polymere Substanz aus einer Komponente, ausgewählt aus der Gruppe Polyurethan, Polyvinylidenfluorid, Polysulfone, Polyethersulfone, Polyester, Poly(meth)acrylate, Butadien-Acrylnitril-Mischpolymere, Polyamide, Polyether/Polyamid-Blockmischpolymere und Ethylen-Vinylalkohol-Mischpolymere, besteht.

**19.** Verfahren nach Anspruch 17, wobei die polymere Substanz aus Polyurethan besteht.

**20.** Verfahren nach Anspruch 17, wobei der Porenbildner aus einer wasserlöslichen Verbindung besteht.

**21.** Verfahren nach Anspruch 20, wobei der Porenbildner aus einem wasserlöslichen Polymer besteht.

**22.** Verfahren nach Anspruch 20, wobei der Porenbildner aus einer Komponente, ausgewählt aus der Gruppe Polyvinylalkohol, Polyvinylpyrrolidon, Methylcellulose, Polyether, Polysaccharide, Polyacrylsäure und deren Salze und Polyacrylamide, besteht.

**23.** Verfahren nach Anspruch 17, wobei das poröse Substrat aus einem Vlies besteht.

## Revendications

**1.** Matériau filtrant capturant les leucocytes constitué par un matériau poreux continu à réseau tridimensionnel comprenant au moins une substance polymérique, lequel matériau filtrant possède un diamètre moyen des pores situé dans le domaine de 1 à 60 $\mu$m, une surface spécifique située dans le domaine de 0,5 à 10 m$^2$/g, une porosité située dans le domaine de 30 à 95 %, un point de bulle situé dans le domaine de 0,08 à 0,40 kg/cm$^2$ et une épaisseur de paroi située dans le domaine de 0,3 à 9,0 mm, et qui est stérilisable avec de la vapeur haute pression.

**2.** Matériau filtrant selon la revendication 1, dans lequel ladite substance polymérique est un élément choisi dans le groupe consistant en le polyuréthane, le poly(fluorure de vinylidène), les polysulfones, les polyéthersulfones, les polyesters, les poly(méth)acrylates, les copolymères butadiène-acrylonitrile, les polyamides, les copolymères séquencés polyéther-polyamide et les copolymères éthylène-alcool vinylique.

**3.** Matériau filtrant selon la revendication 1, dans lequel ladite substance polymérique est le polyuréthane.

**4.** Matériau filtrant selon la revendication 1, dans lequel ledit diamètre moyen des pores est situé dans le domaine de 2 à 50 $\mu$m, ladite surface spécifique est située dans le domaine de 1 à 5 m$^2$/g, ladite porosité est située dans le domaine de 50 à 95 %, ledit point de bulle est situé dans le domaine de 0,13 à 0,27 kg/cm$^2$ et ladite épaisseur de paroi est située dans le domaine de 0,5 à 5,0 mm.

5. Matériau filtrant selon la revendication 1, dans lequel une couche du matériau poreux continu à réseau tridimensionnel est formée sur la surface d'un substrat poreux, lequel matériau filtrant a un point de bulle situé dans le domaine de 0,08 à 0,30 kg/cm$^2$ et un taux de pores superficiels situé dans le domaine de 6 à 90 %.

6. Matériau filtrant selon la revendication 5, dans lequel ladite substance polymérique est un élément choisi dans le groupe consistant en le polyuréthane, le poly(fluorure de vinylidène), les polysulfones, les polyéthersulfones, les polyesters, les poly(méth)acrylates, les copolymères butadiène-acrylonitrile, les polyamides, les copolymères séquencés polyéther-polyamide et les copolymères éthylène-alcool vinylique.

7. Matériau filtrant selon la revendication 5, dans lequel ladite substance polymérique est le polyuréthane.

8. Matériau filtrant selon la revendication 5, dans lequel ledit diamètre moyen des pores est situé dans le domaine de 2 à 50 $\mu$m, ladite surface spécifique est située dans le domaine de 1 à 5 m$^2$/g, ledit point de bulle est situé dans le domaine de 0,13 à 0,27 kg/cm$^2$, ladite épaisseur de paroi est située dans le domaine de 0,3 à 5,0 mm et ledit taux de pores superficiels est situé dans le domaine de 10 à 85 %.

9. Matériau filtrant selon la revendication 5, dans lequel ledit substrat poreux est un non-tissé.

10. Procédé de production d'un matériau filtrant capturant les leucocytes selon la revendication 1 qui comprend la préparation d'une composition de matières premières comprenant au moins une substance polymérique, un bon solvant pour ladite substance polymérique et au moins un agent porogène soluble ou gonflable dans un mauvais solvant pour ladite substance polymérique et compatible avec ledit bon solvant, le moulage par extrusion de ladite composition de matières premières en une forme prédéterminée avec un dispositif d'extrusion puis l'introduction et l'immersion de la composition moulée par extrusion dans un bain constitué principalement par ledit mauvais solvant pour gélifier ladite composition moulée par extrusion et, simultanément, permettre audit agent porogène d'être dissous dans ledit mauvais solvant et par conséquent réaliser son retrait de ladite composition façonnée.

11. Procédé selon la revendication 10, qui comprend en outre l'application d'un revêtement de ladite composition moulée par extrusion sur la surface d'un substrat et l'introduction et l'immersion du substrat revêtu dans ledit bain constitué principalement par ledit mauvais solvant pour gélifier ou réaliser la solidification du revêtement appliqué et, simultanément, permettre audit agent porogène d'être dissous dans ledit mauvais solvant et par conséquent réaliser son retrait dudit substrat revêtu.

12. Procédé selon la revendication 10 ou la revendication 11, dans lequel ladite substance polymérique est un élément choisi dans le groupe consistant en le polyuréthane, le poly(fluorure de vinylidène), les polysulfones, les polyéthersulfones, les polyesters, les poly(méth)acrylates, les copolymères butadiène-acrylonitrile, les polyamides, les copolymères séquencés polyéther-polyamide et les copolymères éthylène-alcool vinylique.

13. Procédé selon la revendication 10 ou la revendication 11, dans lequel ladite substance polymérique est le polyuréthane.

14. Procédé selon la revendication 12, dans lequel ledit agent porogène est un composé hydrosoluble.

15. Procédé selon la revendication 12, dans lequel ledit agent porogène est un polymère hydrosoluble.

16. Procédé selon la revendication 14, dans lequel ledit agent porogène est un élément choisi dans le groupe consistant en le poly(alcool vinylique), la polyvinylpyrrolidone, la méthylcellulose, les polyéthers, les polysaccharides, le poly(acide acrylique) et ses sels, et les polyacrylamides.

17. Procédé selon la revendication 11, dans lequel ledit substrat est poreux, ledit bain d'immersion contient en outre un bon solvant pour la substance polymérique et dans lequel ledit substrat revêtu immergé se solidifie avant la dissolution de l'agent porogène dans le mauvais solvant.

**18.** Procédé selon la revendication 17, dans lequel ladite substance polymérique est un élément choisi dans le groupe consistant en le polyuréthane, le poly(fluorure de vinylidène), les polysulfones, les polyéthersulfones, les polyesters, les poly(méth)acrylates, les copolymères butadiène-acrylonitrile, les polyamides, les copolymères séquencés polyéther-polyamide et les copolymères éthylène-alcool vinylique.

**19.** Procédé selon la revendication 17, dans lequel ladite substance polymérique est le polyuréthane.

**20.** Procédé selon la revendication 17, dans lequel ledit agent porogène est un composé hydrosoluble.

**21.** Procédé selon la revendication 20, dans lequel ledit agent porogène est un polymère hydrosoluble.

**22.** Procédé selon la revendication 20, dans lequel ledit agent porogène est un élément choisi dans le groupe consistant en le poly(alcool vinylique), la polyvinylpyrrolidone, la méthylcellulose, les polyéthers, les polysaccharides, le poly(acide acrylique) et ses sels, et les polyacrylamides.

**23.** Procédé selon la revendication 17, dans lequel ledit substrat poreux est un non-tissé.

# FIG.1

# FIG.2

# FIG.3